# EUROPEAN PATENT APPLICATION

(11) **EP 1 300 404 A1**
(43) Date of publication of application: **09.04.2003**
(21) Application number: 01947931.0
(22) Date of filing: 09.07.2001
(51) Int. Cl.: C07D 311/36, A61K 31/352, A61K 35/78, A61P 3/04, A23L 1/30

(54) **OBESITY INHIBITORY MATERIALS**

(30) Priority: 07.07.2000 JP 2000207422
(71) Applicant: Nichimo Co., Ltd, Tokyo 140-0002 (JP)
(72) Inventor: TAKEBE, Minoru, c/o Nichimo Co., Ltd, Tokyo 140-0002 (JP)
(74) Representative: Ouzman, Beverley Nicola Claire
(86) International application number: JP0105944
(87) International publication number: WO02004437

(57) **Abstract**

Anti-obesity material of the present invention is absorbed *in vivo* by oral ingestion, drops, etc. so as to act against the causes of overeating and control food consumption, and it can thereby promote fat metabolism and control accumulation of body fat while at the same time can inhibit elevation of blood pressure, all without compromising immune system function, thus safely controlling an increase in body weight (obesity). There have previously been no anti-obesity materials like that of the present invention whatsoever. The main characteristic of the present invention is that an anti-obesity material having the above-described excellent effects is obtained since the anti-obesity material has isoflavone aglycone and/or isoflavone glycoside.

## Description

### Technical Field

The present invention relates to an anti-obesity material capable of controlling obesity (weight increase) and in particular to an anti-obesity material capable of effectively controlling obesity by controlling an increase in body weight while controlling food consumption and enhancing *in vivo* immune function as a result of being absorbed *in vivo*.

### Background Art

Obesity is a cause of development of lifestyle-related disease, including hypertension, diabetes, hyperlipidemia, etc. Moreover, it is reported that, according to epidemiological research, when obese persons lose weight, their average remaining years are improved. However, it is also a fact that even though obese persons recognize the fact that they are at high risk for lifestyle-related disease, they do not lose weight because it is extremely difficult for them to keep weight off by dietary, etc. methods.

Postmenopausal women in particular develop so-called climacteric symptoms due to a reduction in female hormone (estrogen) secretion. In addition to the climacteric disturbances represented by hot flashes, these symptoms are said to have an effect on general functions of bone metabolism, the cardiovascular system, fat metabolism, the urogenital system, the digestive system, the neurological system, etc. Consequently, hormone-replacement therapy (HRT) whereby female hormones are artificially replenished is being examined as a treatment for the symptoms caused by this loss of estrogen activity. Nevertheless, HRT also poses many problems in that adverse reactions such as uterine bleeding and breast tenderness, etc. readily develop.

Obesity in women not only is affects appearance but is also a factor that increases the risk of lifestyle-related disease. Furthermore, although it is clear that obesity readily develops after menopause, it is also true that there are no means for coping with the obesity. Obesity is a result of advanced storage of energy in the form of fat cells due to an increase in food consumption (overeating). Therefore, it is necessary to identify the causes of overeating and prevent overeating.

Nevertheless, the dietary methods being used today often involve compulsory control of food consumption while expending the energy that has been stored by intense exercise, but there is a major problem in that obesity is further increased by rebound. Moreover, controlling overeating poses a problem in that there is a chance of disturbing the balance of nutrient consumption, leading to compromising the immune system and increasing the risk of infection from infectious disease and inducing cancer.

Several materials that promote decomposition of fat cells have been presented (Japanese Patent Nos. 2,829,387 and 2,829,388 as well as Japanese Patent Application Laid-Open (Kokai) No. H11-228430). However, these proposals give no disclosure whatsoever of controlling food consumption or enhancing estrogen activity and decomposing fat. Furthermore, these reports describe the results that are based on experiments on the cellular level, and these reports are confirmed by laboratory animal experiments.

Arjmandi et al (Bahram, H., Arjmandi et al., Nutrition Research Vol. 17, No. 5, pp 885-894 (1997)) reports that they were able to lower blood cholesterol and body fat by adding soy protein with soy isoflavones and ipriflavone in experiments using rats on which oophorectomy had been performed. However, the extent of the reduction in comparison to when nothing was added was small, and it could not be confirmed that there was obvious control of an increase in body weight.

### Disclosure of Invention

The present invention was made in light of these points. The object is to present an anti-obesity material that is absorbed *in vivo* by oral ingestion, drops, etc. to act against the causes of overeating and control food consumption, and that can thereby promote fat metabolism and control accumulation of body fat while at the same time can also inhibit elevation of blood pressure, all without compromising immune system function, and thus safely control an increase in body weight (obesity).

As a result of intense research, the inventors completed the present invention upon discovering that by absorbing isoflavone aglycone and isoflavone glycoside (isoflavone compounds) *in vivo*, food consumption is controlled, while immune function is enhanced rather than compromised, and therefore, control of obesity can be expected.

Beatty W.W. et al. (Beatty, W. W., O'Briant, D.A., Vilberg, T. R., *Pharma. Biochem., and Behavior.*, 3:539 (1975)) report that food consumption by rats on which oophorectomy has been performed is accelerated and body weight increases when compared to rats on which oophorectomy has not been performed (sham) in experiments using rats on which oophorectomy has been performed as climacteric model laboratory animals, and Yoshida et al. (Yoshida, T., Nishioka, H., Yoshioka, K., Kondo, M.: Metabolism, 36, 1, 1987) report that food consumption is controlled and body weight is reduced by estrogen administration. One factor in this could be that food consumption is increased because, when compared to the sham rats, secretion of norepinephrine, which is a type of catecholamine, was not reduced by activation of the sympathetic nervous system among the oophorectomy rats. Moreover, it is clear that norepinephrine secretion is reduced by administration of estrogen. This indicates that norepinephrine is not reduced in oophorectomy rats because there is a reduction in internal secretion of estrogen, and therefore, there is a reduction in bonding of estrogen with estrogen receptors of the ventromedial nucleus of the hypothalamus (VMH). Furthermore, it also appears that tyrosine-3-hydroxylase, which is an enzyme that synthesizes norepinephrine, rises with age and that norepinephrine therefore also rises. In any case, it is possible to administer estrogen in order to reduce the food consumption that is a factor in obesity among postmenopausal women, etc., but adverse effects readily develop by this method, posing a problem in terms of safety.

Nevertheless, isoflavones have weak estrogen-like activity. When isoflavones are taken, they bind with estrogen receptors. As a result, isoflavones function in place of estrogen, the internal secretion of which is reduced in post-menopausal women, to reduce the norepinephrine at the VMH.

Furthermore, Aoyama et al. (Aoyama, M. et al., (1998), J. Reprod. Dev., 44:141-148) report that the reduction in food consumption during estrus was due to estrogen in experiments with Shiba goats. On the other hand, Okamura et al. (Okamura, H. et al., (1994), Endocrinology, 135:1705-1708) thought that because estrogen activity is realized via its specific receptor (ER), the site of this ER is a target organ of estrogen. ER is widely distributed in peripheral organs, including the uterus and mammary grand, as well as the brain, and particularly large amounts are present in the VMH. Therefore, the VMH is probably one of the satiation centers that participate in control of eating behavior. Accordingly, it is clear that nitrogen monoxide (NO)-synthesizing enzymes are also present in the majority of nerve cells having the ER within the nerve nucleus and estrogen acts directly to markedly increase the production of NO. Morley et al. (Morley, J. E. et al., (1995), Pharmacol. Biochm. Behav., 50:369-373) proved through laboratory animal experiments that NO-synthesizing enzyme acts to control food consumption without inducing an uncomfortable feeling or onset of disease. Furthermore, Aoyama et al. (Aoyama, M. et al., (1998); J. Reprod. Dev., 44:149-159) studied the mode of action of estrogen relating so as to control of eating behavior focusing on ER and NO of the VMH and discovered that even if the blood estrogen concentration is high, there is not a reduction in food consumption when NO synthesis is inhibited, and therefore, there is a strong chance that the ER-NO system plays some type of role. Thus, in the present invention, it is believed that isoflavones, which are confirmed to have estrogen-like activity, react with NO to bring the obesity center of the VMH to normal.

Furthermore, it is a known fact that appetite is suppressed with an increase in serotonin in the VMH, and this serotonin is also known to be activated when tryptophan is consumed. It appears that isoflavone aglycone functions so that tryptophan is easily taken up *in vivo*.

Nevertheless, thus far there have been no studies on controlling obesity by way of administration of isoflavone from the point of preventing obesity. The inventors completed the present invention upon discovering that by the administration of isoflavone to oophorectomy rats food consumption can be controlled and as a result, there is control of an increase in body weight. Furthermore, the inventors completed the present invention upon discovering that an increase in body weight and a rise in blood pressure can be inhibited by administering isoflavones to male stroke-prone spontaneously hypertensive rats (SHRSP). It became clear from these facts that isoflavones act to produce NO and control a rise in blood pressure, as well as act to promote fat metabolism, etc., and thereby have body weight-controlling effects in women, as well as men. Moreover, it can be said that estrogen receptors (ER) are present in central nerves as well as the vascular endothelium in both men and women and isoflavones act with these ER.

The anti-obesity material of the present invention as described above is characterized in that it contains isoflavone aglycone and/or isoflavone glycoside.

When the anti-obesity material that contains isoflavone aglycone or isoflavone glycoside of the present invention is absorbed *in vivo,* food consumption is controlled, while immune function is enhanced rather than compromised. Thus, safe control of obesity is expected.

The isoflavone aglycone and isoflavone glycoside are preferably materials derived from grains. Moreover, pulse crops are particularly good as materials derived from grains. The isoflavone aglycone is preferably produced by way of fermentation of pulse crops by koji mold in order to hydrolyze the components of the pulse crops by the enzymes of the koji mold. Furthermore, isoflavone aglycone and isoflavone glycoside obtained as described above can also be produced by extraction and concentration by a solvent. The isoflavone aglycone in this case is preferably brought to a daizein content of at least 70 wt%.

In addition, the anti-obesity material of the present invention can be made into an oral supplement so that it can be easily taken orally and safely absorbed by the digestive tract.

### Brief Description of Drawings

Figure 1 is a diagram of the processes that produce the anti-obesity material of the present invention,
Figure 2 is a graph showing changes in food consumption by rats, etc., that had ingested the anti-obesity material of the present invention,
Figure 3 is a graph showing changes in weight of rats, etc., that had ingested the anti-obesity material of the present invention,
Figure 4 is a graph showing changes in weight of rats, etc., that had ingested the anti-obesity material of the present invention,
Figure 5 is a graph showing changes in spleen weight after radiation exposure of rats, etc., that had ingested the anti-obesity material of the present invention,
Figure 6 is a graph showing changes in spleen weight after radiation exposure per 100 g body weight of rats, etc., that had ingested the anti-obesity material of the present invention,
Figure 7 is a graph showing the number of spleen colonies formed following marrow transplant after radiation exposure of rats, etc., that had ingested the anti-obesity material of the present invention,
Figure 8 is a graph showing changes in body weight of SHRSP rats, etc., that had ingested the anti-obesity material of the present invention,
Figure 9 is a graph showing changes in food consumption of SHRSP rats, etc., that had ingested the anti-obesity material of the present invention,
Figure 10 is a graph showing changes in systolic blood pressure of SHRSP rats, etc., that had ingested the anti-obesity material of the present invention,
Figure 11 is a graph showing changes in urine NO₂/NO₃ of SHRSP rats, etc., that had ingested the anti-obesity material of the present invention,
Figure 12 is a graph showing changes in adrenaline of the urine catecholamines in SHRSP rats, etc., that had ingested the anti-obesity material of the present invention,
Figure 13 is a graph showing changes in noradrenaline of the urine catecholamines of SHRSP rats, etc., that had ingested the anti-obesity material of the present invention,
Figure 14 is a graph showing changes in dopamine of the urine catecholamines of SHRSP rats, etc., that had ingested the anti-obesity material of the present invention,
Figure 15 is a graph showing changes in serum total cholesterol, HDL-cholesterol, LDL-cholesterol, and the cholesterol ester ratio of SHRSP rats, etc., that had ingested the anti-obesity material of the present invention,
Figure 16 is a graph showing changes in serum triglycerides of SHRSP rats, etc., that had ingested the anti-obesity material of the present invention,
Figure 17 is a graph showing changes in free fatty acids of SHRSP rats, etc., that had ingested the anti-obesity material of the present invention, and
Figure 18 is a graph showing changes in visceral fat of SHRSP rats, etc., that had ingested the anti-obesity material of the present invention.

### Best Mode for Carrying Out the Invention

Embodiments of the present invention will be described below.

The anti-obesity material of the present invention is characterized in that it contains isoflavone aglycone and/or isoflavone glycoside. More specifically, the anti-obesity material of the present invention is food and drugs in solid (including all forms, such as powder, granules, etc.) or liquid form that contain isoflavone aglycone only (pure substance) and isoflavone glycoside only (pure substance) or at least one of these isoflavones.

The isoflavone aglycone and isoflavone glycoside can be obtained from any starting material; and it is preferred that the isoflavone aglycone and isoflavone glycoside be a material derived from cereals obtained using grains as the starting material. Moreover, it is particularly preferred that the grain-derived material be produced by fermentation of pulse crops by koji mold and then by hydrolysis so as to decompose the components of the pulse crops by the enzymes of the koji mold. It is further preferred that the material produced by the hydrolysis be further extracted and concentrated by a solvent to produce isoflavone aglycone.

When the anti-obesity material with isoflavone aglycone or isoflavone glycoside of the present invention is absorbed *in vivo* continuously by through oral administration or drops over a specific period of time, food consumption is controlled, while immune function is enhanced rather than compromised, and an increase in body weight is thereby controlled and safe control of obesity is expected.

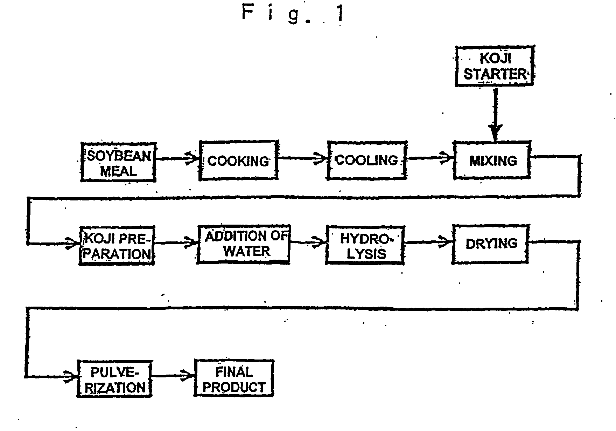

Next, the manner of producing the anti-obesity material of the present invention, that contains isoflavone aglycone and isoflavone glycoside, by way of fermentation using koji mold will be described with reference to Figure 1.

Figure 1 is one example of the method of producing the anti-obesity material of the present invention from soybean meal, which is a pulse crop, a type of grain.

The manner of producing the anti-obesity material of the present invention will be described in accordance with the processes shown in Figure 1. First, the soybean meal is cooked. Propagation of the soybean meal is facilitated by this cooking when compared to when cooking is not performed. Depending on the purpose of production, etc, cooking of the soybean meal is performed by the batch method, whereby cooking and subsequent koji preparation are separately performed, or by the continuous method using a koji preparation device with which cooking and subsequent koji preparation are continuously performed.

Once cooking has been completed, the water content of the soybean meal is adjusted to the content with which propagation of koji mold can be accomplished (for instance, approximately 36 wt%).

The stem cell-augmenting material of the present invention is produced in the following manner from soybean meal whose water content has been adjusted in this way.

More specifically, when the cooked soybean meal simple substance is cooled to bring its temperature to 40°C or lower so as not to kill the koji mold, the soybean meal is inoculated with koji starter consisting of koji mold to a specific weight ratio, and then these two are mixed until a uniform mixture is obtained.

So as to raise the temperature of the mixture as a result of propagation of the koji starter, the temperature of the mixture is once reduced inside a koji preparation device and the mixture is set aside while keeping temperature at approximately 32°C. As a result, the soybean meal is fermented by the koji mold, and temperature rises to approximately 38°C as koji preparation thus proceeds; and the product becomes firm and solidifies as hyphae of the propagating koji mold grow. Then, as koji preparation proceeds, the solidified product is released from the koji preparation device by being spun and agitated. Ventilation is provided in order to uniformly feed air to the inside and control temperature so that product temperature is reduced to approximately 33 - 35°C. Koji preparation proceeds further as ventilation is continued. As a result, death of the koji mold is reliably prevented, and propagation of koji mold is satisfactorily accomplished. Then, the product inside the koji preparation device is spun and agitated enough times depending on how firmly the product has solidified inside the koji preparation device as ventilation is continued.

The koji mold used for the above koji preparation is the koji mold that has been used for many years in fermented food products unique to Japan and "tempe." The genus *Aspergillus,* including *Aspergillus usamii, Aspergillus kawachii, Aspergillus awamori, Aspergillus saitoi, Aspergillus oryzae, Aspergillus niger,* etc., and those classified as the genus *Rhizops,* which are safe for food products, are preferably used.

The fermentation time is at least 24 hours or longer, depending on the species of koji mold that is used. The fermentation time should be long enough to thoroughly propagate the koji and decompose a specific amount of proteins and/or saccharides in the soybean meal.

In this embodiment, hydrolysis of the proteins and/or saccharides is further performed on the product of koji preparation. Here, the term saccharides includes saccharides such as sucrose, etc. and starches etc.

Hydrolysis after this koji preparation treatment is also performed while keeping the substrate as a flake state. In other words, in this embodiment, water is added to the product after completing koji preparation to bring the water content to, for instance, 50 wt%, and then the product (resultant) is heated to 30 - 45°C, and temperature is kept constant for a specific time in order to hydrolyze the proteins and/or carbohydrates contained in the product of koji preparation treatment. The proteins and/or carbohydrates are thus hydrolyzed, and large amounts of isoflavone aglycone and isoflavone glycoside with anti-obesity performance are produced.

Specific examples of the anti-obesity material of the present invention will be described next.

In the above fermented soybean meal, which becomes the anti-obesity material that is produced by the present invention using *Aspergillus oryzae* as the koji mold as described above, the isoflavone compounds have been thoroughly decomposed. In other words, although untreated soybean meal contains relatively large amounts of daizin and genistin, which are glycoside, when compared to daizein and genistein, which are aglycones, the fermented soybean meal produced by the present invention contains very little diazin and genistin, or glycoside, because they have been decomposed, and contains a very large amount of daizein and genistein, or aglycones, that are produced by decomposition. Table 1 compares the isoflavone compound content of untreated soybean meal with that of soybean meal obtained by adjusting product temperature to 35°C while ventilating, performing koji preparation for 48 hours, adding water to a water content of approximately 50%, and then setting aside for 24 hours or longer at 45°C to allow the soybean components and koji mold components to be hydrolyzed by the enzymes in the koji mold. It is clear that large amounts of daizein and genistein, which are the aglycone form of isoflavone compounds, are obtained.

**Table 1**

| | | | |
|---|---|---|---|
| Daizin | Daizein | Genistin | Genistein |
| Not detected | 70 | 103 | 64 |
| (Units: mg/100 g) | | | |

Soybean embryo will be described below with reference to the present invention. After soybean embryo was pre-steamed and then water was added to bring the water content to approximately 37%, the resultant was steamed, sterilized, and cooled; and then with respect to the mixture ratio of soybean embryo and koji mold, 200 g inoculum that had been adjusted to 8 x 10⁷ koji mold spores/g (adjusted with polished rice) were mixed per 400 kg soybean embryo. Furthermore, after cooling the resultant to 32°C when koji preparation was started, koji preparation proceeded without ventilation until the product temperature reached 40°C; and when temperature reached 40°C, the koji preparation proceeded with an increase in temperature being prevented while providing ventilation. The first agitation ("mori" process) was conducted for approximately 17 hours after starting of koji preparation. The soybean embryo gave off heat, and temperature was controlled while providing ventilation so that the product temperature after agitation would be approximately 35°C. Next, the second agitation ("naka" process) was performed after approximately eight hours, and heat was given off. The product temperature was again controlled to approximately 35°C by ventilation, and the third agitation ("shimai" process) was further performed as in the previous process after approximately 16 hours. Then, koji preparation was completed, approximately 48 hours after starting, by controlling temperature while providing ventilation so that the product temperature would be approximately 38°C. Once koji preparation was completed, water content of the product was adjusted while agitating it to bring the water content to 50%, and the product was heated to a temperature of approximately 50°C. Then, the majority of the isoflavone compounds in the soybean embryo were hydrolyzed to aglycone form by the koji mold enzymes for 48 hours or longer. Isoflavone compounds of the soybean embryo obtained by treatment of the present invention were obtained in large quantities, mainly in the aglycone form of daizein, as shown in Table 2.

**Table 2**

| Units: mg/100 g | | | |
|---|---|---|---|
| Components | | Present invention | Untreated |
| Glycoside | Daizin | 70 | 840 |
| | Genistin | 40 | 170 |
| | Glycitin | 130 | 620 |
| | Total | 240 | 1630 |
| Aglycone | Daizein | 680 | 10 |
| | Genistein | 130 | 2.4 |
| | Glycitein | 240 | 2.0 |
| | Total | 1050 | 14.4 |

Moreover, it is preferable that the product obtained by hydrolysis be further extracted and concentrated using solvent in the present embodiment to obtain a concentrated product of 30 wt% or more isoflavone aglycone, as shown in Table 3.

**Table 3**

| Composition of Concentrated Product of Material of the Present Invention | | |
|---|---|---|
| Analysis items | Standard value | Analytical value |
| Properties | Pale yellow powder with no offensive taste, offensive odor or impurities | Passed |
| Weight loss on drying | 5% or less | 2.8% |
| Ignition residue | 3% or less | 0.1% |
| Arsenic | 2 ppm or less | Passed |
| Heavy metals | 20 ppm or less | Passed |
| Agricultural chemicals residue | Not detected | Not detected |
| General number of live bacteria | 3000 bacteria/g or less | 3000 bacteria/g or less |
| *Escherichia coli* group | Negative | Negative |
| Amount of isoflavone aglycone | 30% or more | 32.9% |
| Daizein | | 23.4% |
| Glycitein | | 8.0% |
| Genistein | | 1.5% |

Examples of the present invention will now be described:

### Example 1

In order to conduct experiments on physical constitutions predisposed to gaining weight, experiments were performed on rats on which oophorectomy had been performed. SD rats were used in the experiments.

### a) Experimental method

1) Oophorectomy rats (nine weeks old) and sham rats on which sham surgery had been performed without excising ovaries (nine weeks old) were acclimated for one week or longer and healthy female rats of the same body weight in each group were reared as the samples in order to evaluate as well the difference due to oophorectomy. By using feed with the composition shown in Table 4 during this rearing, the effect of obesity control by feed composition was eliminated. In other words, the protein source of this feed was the animal protein casein. This casein is a protein that predisposes to obesity, and the effect of obesity control by feed composition was eliminated by using this protein.

**Table 4**

| Feed composition | g/100g |
|---|---|
| Casein | 22.70 |
| Sucrose | 41.76 |
| Cornstarch | 20.00 |
| Cellulose | 5.60 |
| Corn oil | 5.70 |
| Vitamin mix | 1.00 |
| Vitamin D3 | 0.00016 |
| Mineral mix | 1.34 |
| Calcium carbonate | 0.988 |
| Sodium phosphate | 0.388 |
| Potassium phosphate | 0.238 |
| Potassium citrate | 0.090 |

2) Carrier prepared to a concentration of 0.5 wt% by dissolving Japan Pharmacopoeia carmellose sodium in Japan Pharmacopoeia water for injection and suspensions of known concentrations of two types of concentrated materials of isoflavone aglycone and isoflavone glycoside mixed in this solvent were used as the substances administered to the rats.
3) The animals were grouped eight sham rats in comparative group 1 and 8 oophorectomy rats in comparative group 2 and experimental groups 1, 2 and 3 each. The administration substances shown in Table 5 were orally administered gavage once a day to each rat of each group using a stomach tube, with the ratio to the most recent body weight being 10 ml/kg. The amount of isoflavone aglycone and isoflavone glycoside administered to experimental groups 1, 2 and 3 was adjusted to the dose in Table 5.

**Table 5**

| Administration group | | Administration substance | Dose (mg/kg) | Number of animals |
|---|---|---|---|---|
| Comparative group 1 | Sham surgery | Carrier | -- | 8 |
| Comparative group 2 | Oophorectomy | Carrier | -- | 8 |
| Experimental group 1 | Oophorectomy | Carrier + concentrated material | 200 | 8 |
| Experimental group 2 | Oophorectomy | Carrier + concentrated material | 50 | 8 |
| Experimental group 3 | Oophorectomy | Carrier + isoflavone glycoside | 80 | 8 |

4) Food consumption (Amount of feed consumed)
Two-day food consumption was determined once a week for all rats, and the daily average food consumption was calculated.
5) Determination of body weight
Body weight of all rats was determined on the day administration was started and once a week thereafter.
6) Determination of weight of lateral uterus weight adipose tissue
Weight of the lateral uterus adipose tissue of each rat was determined in terms of adipose tissue (g) and adipose tissue (g%) once the experiment was completed.
7) Statistical treatment of data
Equal variance tests were performed on the quantitative data of each group by the Bartlett method; and when there was equal variance, variance analysis was performed by the 1-way layout method. If there was significance, inter-group comparisons with the comparative group were performed by the Dunnett method. On the other hand, if there was no equal variance, Kruskal-Wallis tests were performed; and if there was significance, inter-group comparison with the comparative group was performed by the Dunnett method using rank. Incidentally, the tests were two-sided tests with the level of significance being 5 and 1%.

### b) Experimental results

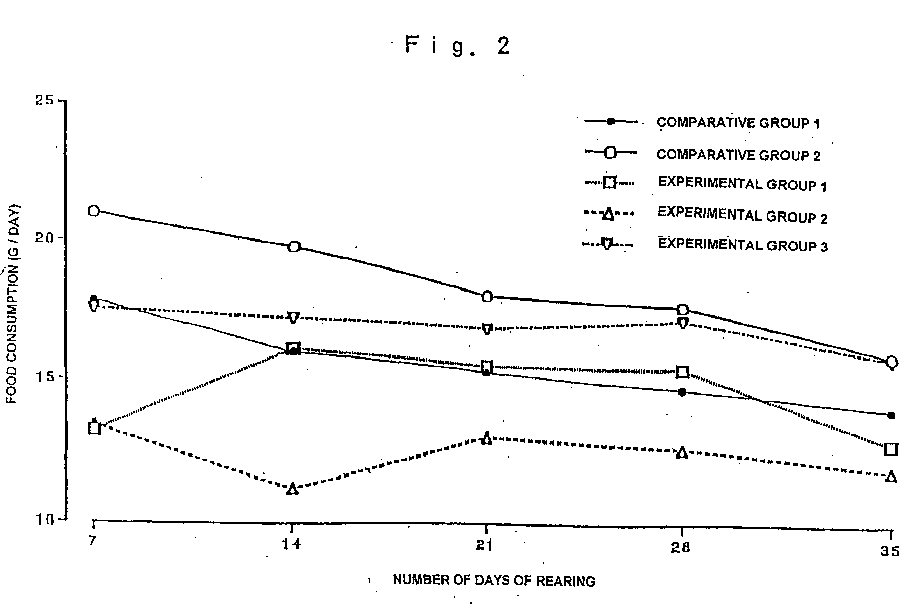

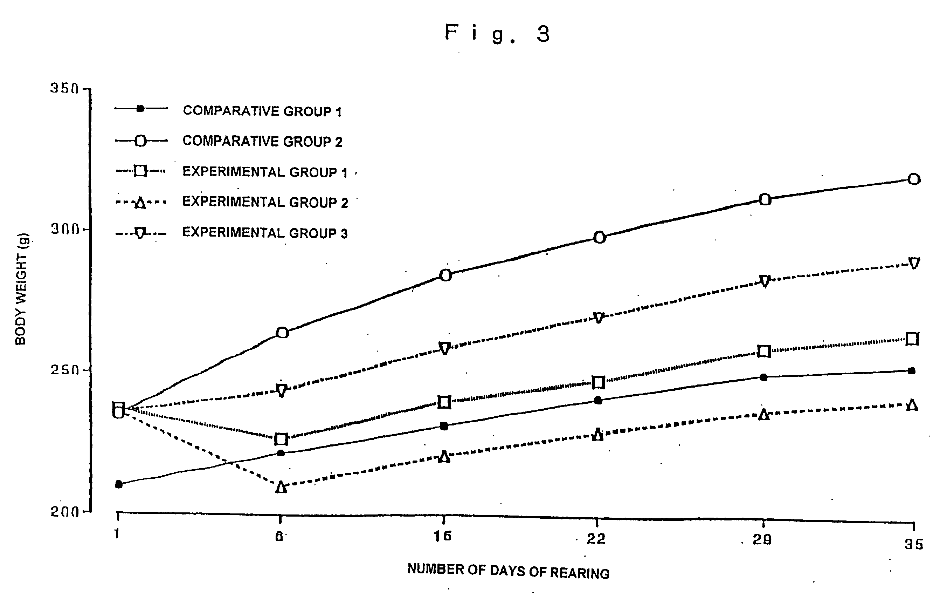

1) The results of determining food consumption are as shown in Table 6 and Figure 2.
2) The results of determining body weight are as shown in Table 7 and Figure 3.

**Table 6**

| Food Consumption | | | | | |
|---|---|---|---|---|---|
| Administration group Number of animals | Comparative group 1 10 | Comparative group 2 10 | Experimental group 1 10 | Experimental group 2 10 | Experimental group 3 10 |
| Number of days 7 | 17.8±1.4t | 21.0±3.0 | 13.2+6.3** | 13.4±5.6** | 17.5±2.9 |
| 14 | 16.0±1.6tt | 19.8±2.7 | 16.1±4.7 | 11.2±3.0**(9) | 17.2±3.6 |
| 21 | 15.3±1.4t | 1.8±2.9 | 15.5±3.8 | 13.0±1.7**(9) | 16.8±2.6 |
| 28 | 14.7±1.9tt | 17.6±1.7 | 15.4±1.2** | 12.6±1.1**(9) | 17.1±1.7 |
| 35 | 14.0±1.1tt | 15.9±1.4 | 12.8±2.6* | 11.9±2.8**(9) | 15.8±2.1 |

| | | | | | |
|---|---|---|---|---|---|
| (): Number of animals t, tt: There was a significant difference at a limit of 0.05 or less and 0.01 or less, respectively, between comparative example 1 and comparative example 2. *,**: There was a significant difference at a limit of 0.05 or less and 0.01 or less, respectively, between comparative group 2 and each experimental group. | | | | | |

**Table 7**

| Body Weight | | | | | |
|---|---|---|---|---|---|
| Administration group Number of animals | Comparative group 1 10 | Comparative group 2 10 | Experimental group 1 10 | Experimental group 2 10 | Experimental group 3 10 |
| Number of days 1 | 209.8±7.2tt | 235.2±7.5 | 236.8±7.3 | 235.9±7.0 | 235.8±8.2 |
| 8 | 222.1±7.6tt | 264.4±11.6 | 227.0±13.9** | 210.2±22.9 ** | 243.9±19.0** |
| 15 | 231.7±9.6tt | 285.0±15.8 | 240.0±7.5 ** | 221.1±14.3** (9) | 258.8±16.1** |
| 22 | 241.6±11.7tt | 299.2±18.0 | 247.6±10.6** | 229.8±12.3**(9) | 270.5±16.7** |
| 29 | 250.0±12.7tt | 312.6±18.3 | 259.2±11.0 ** | 237.2±14.6**(9) | 284.2±19.9** |
| 35 | 253.5±10.8tt | 321.1±18.1 | 264.7±14.5** | 241.8±14.0**(9) | 291.7±21.4** |

| | | | | | |
|---|---|---|---|---|---|
| (): Number of animals tt: There was a significant difference at a limit of 0.01 or less between comparative group 1 and comparative group 2. *,**: There was a significant difference at a limit of 0.05 or less and 0.01 or less between comparative group 2 and each experimental group. | | | | | |

3) Determinations of weight of the lateral uterus adipose tissue are as shown in Table 8.

**Table 8**

| Weight of Lateral Uterus Adipose Tissue | | | | | |
|---|---|---|---|---|---|
| | Comparative group 1 | Comparative group 2 | Experimental group 1 | Experimental group 2 | Experimental group 3 |
| Adipose tissue (g) | 4.82±1.56 | 6.70±1.65 | 3.31±1.24** | 1.80±0.61** | 4.14±0.84* |
| Adipose tissue (g%) | 1.98±0.58 | 2.17±0.42 | 1.31±0.46** | 0.78±0.24** | 1.50±0.31** |

### c) Evaluation

It is clear based on changes in food consumption shown in Table 6 and Figure 2 that the anti-obesity function possessed by the anti-obesity material of the present invention came well into effect, keeping an increase in food consumption by rats low and controlling the advancement of obesity.

More specifically, although food consumption was high in the oophorectomy rats (comparative example 2), food consumption when compared to comparative group 2 was kept significantly low by administration of isoflavone aglycone at 200 mg/kg body weight (experimental group 1) and isoflavone aglycone at 50 mg/kg body weight (experimental group 2). Furthermore, the fact that food consumption in experimental group 1 was lower than that of the sham rats (comparative group 1) indicates that isoflavone aglycone has the effect of keeping food consumption low in a dose-dependent manner. Although not significant when compared to comparative group 2, it was clear that food consumption is kept low by isoflavone glycoside administered at 80 mg/kg body weight (experimental group 3). This indicates that isoflavone aglycones have superior effects in terms of reducing food consumption.

Furthermore, oophorectomy rats are climacteric female model animals. Climacteric symptoms accompany menopause and a reduction in the amount of estrogen secreted is a factor. This results in an increase in food consumption. A significant reduction in food consumption of climacteric female model animals when administered the isoflavone concentrated material, that is isoflavone aglycone concentrated material, as shown in experimental groups 1, 2 and 3, was confirmed in this example. Consequently, the anti-obesity function of the anti-obesity material of the present invention is nothing short of keeping food consumption low.

As shown by the changes in body weight in Table 7 and Figure 3, an increase in body weight was controlled in proportion to the reduction in food consumption previously described. It can be said that an increase in body weight is controlled because food consumption is kept low as a result of administering the anti-obesity material of the present invention. Although control of an increase in body weight could be confirmed with both isoflavone glycoside and isoflavone aglycone, this tendency was stronger with isoflavone aglycone and was enhanced with a larger dose. This is nothing short of isoflavone aglycone having excellent absorption *in vivo.*

Comparative group 1 was the sham rats. These rats did not show a reduction in estrogen secretion and were not obese. However, comparative group 2 were oophorectomy rats. These rats did show a reduction in estrogen secretion. There was a tendency toward an increase in food consumption and obesity. Consequently, it was possible to check whether or not body weight increased in comparison to comparative group 2 by administering the anti-obesity material of the present invention. Moreover, it appears that if the sham rats (comparative group 1) are regarded as rats of normal body weight, body weight should be the same as that of the sham rats (comparative group 1). Lower than this body weight is also regarded as a tendency toward weight loss. When evaluated from this point, an increase in body weight when compared to comparative group 2 was controlled in experimental groups 1, 2 and 3. When explained in further detail, it can be said that experimental group 1 changed by almost the same body weight changes as in the sham rats (comparative group 1). An increase in body weight was further controlled in experimental group 2 when compared to the sham rats (comparative group 1). This indicates that the anti-obesity material of the present invention, that is, isoflavone aglycone concentrated product, can enhance obesity control by adjusting the dose.

The determination results in Table 8 show the state of accumulation of body fat of rats in each group. It is a known fact that body fat accumulates with obesity. The main component of fat is neutral fat, and it is said that changes in the lateral uterus adipose tissue weight that was determined correspond to general changes in neutral fat content of the rat (Rizack, M. A.: J. Bio. Chem., 236-657 (1961)). Consequently, it is clear from the results in Table 8 that there is little *in vivo* accumulation of neutral fat in experimental groups 1, 2 and 3. It is also lower than in sham rats (comparative group 1), making it clear that accumulation of neutral fats is efficiently controlled by ingesting the anti-obesity material of the present invention.

### Example 2

Radiation exposure and the mouse spleen colony method accompanied by marrow transplant were conducted in rats in order to perform experiments on the correlation between control of obesity and *in vivo* immune function in the present example.

### a) Test method

Healthy female rats of the same body weight in each group were reared as the samples (female ICR) (eight weeks old).
2) Ordinary food and feed where isoflavone aglycone concentrated material and isoflavone glycoside were mixed with this ordinary food were used as the substances administered to rats.
3) The rats were divided into comparative groups 1 and 2 and an experimental group with five animals each. As shown in Table 9, ordinary food was administered to comparative groups 1 and 2, while isoflavone aglycone was added to ordinary food at a ratio of 30 mg/kg body weight and administered to the experimental group. Each group was reared for three weeks.

**Table 9**

| Administration group | | | Administration substance | Dose (mg/kg) | Number of animals |
|---|---|---|---|---|---|
| Comparative group 1 | Unexposed | Marrow transplant | Ordinary food | -- | 5 |
| Comparative group 2 | Radiation exposure | Marrow transplant | Ordinary food | -- | 5 |
| Experimental group 1 | Radiation exposure | Marrow transplant | Ordinary food + concentrated material (0.1%) | Isoflavone aglycone 30 mg/kg | 5 |

4) Radiation exposure
The rats of comparative group 2 and the experimental group were systemically exposed one time only to a dose of 8 Gy after three weeks of rearing.
5) Bone marrow transplant
Bone marrow transplant was performed on the rats of each group 24 hours after radiation exposure. In this case, the femur of a donor rat of the same species was cut out. A small piece was cut off from both ends of the femur with a clean bench. Once 1 ml RPMI medium 1640 medium containing 10% antibiotic-antimycotic was injected with a syringe from one end through the medullary cavity, it was pushed into a test tube. The marrow cell count was adjusted to 10⁵/ml, and 0.5 ml was injected from a caudal vein. The donor rat had been systemically exposed to 2 Gy radiation and reared for four weeks on ordinary food.
6) Determination of body weight
Body weight of all rats was determined before bone marrow transplant and seven days after bone marrow transplant
7) Determination of spleen weight
All rats were sacrificed after determination of body weight seven days after the bone marrow transplant. The spleen was excised, and the spleen weight and number of spleen colonies formed were determined.

### b) Experimental results

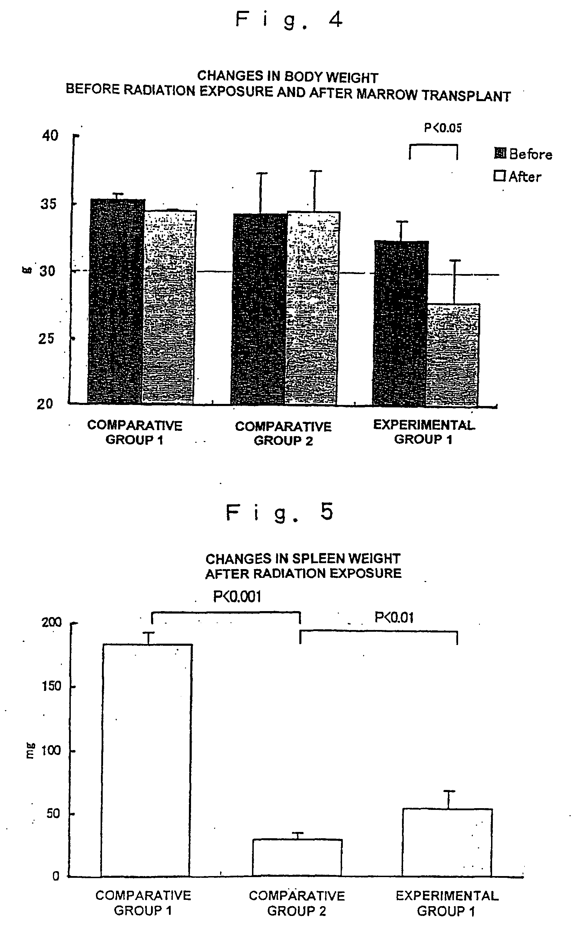

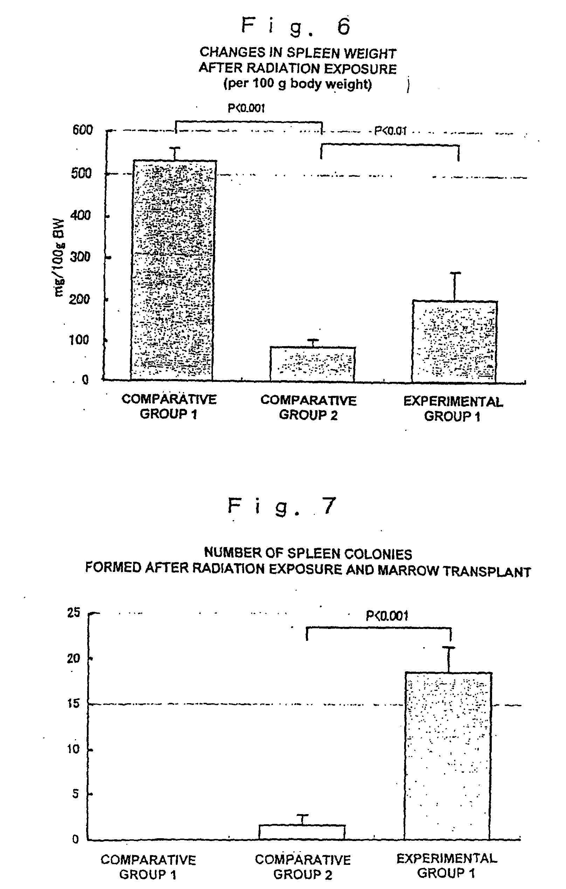

1) The results of measured body weight are as shown in Figure 4.
2) The changes in spleen weight after exposure to radiation are as shown in Figure 5, the changes in spleen weight after exposure per 100 g body weight are as shown in Figure 6, and the number of spleen colonies that formed after marrow transplant following radiation exposure are as shown in Figure 7.

### c) Evaluation

Based on the changes in body weight shown in Figure 4, rats inoculated with isoflavone aglycone of the present invention in the experimental group showed significant control of an increase in body weight, even before radiation exposure, when compared to the other groups, and even greater control of an increase in body weight after radiation exposure when compared to the other groups.

It is clear based on changes in body weight shown in Figure 4 that the anti-obesity function possessed by the anti-obesity material of the present invention came well into effect to control the advancement of obesity.

Based on the experimental results relating to the spleen shown in Figures 5 through 7, when the other comparative group 2 and the experimental group are seen in comparison to rats not exposed to radiation in comparative group 1, the increase in spleen weight is low and further the number of spleen colonies formed is also low and therefore *in vivo* immunity is compromised in comparative group 2 that did not ingest the anti-obesity material of the present invention; on the other hand, the increase in spleen weight is large and further the number of spleen colonies formed is also high and therefore *in vivo* immunity is significantly enhanced in the experimental group that did ingest the anti-obesity material of the present invention.

In other words, when the anti-obesity material having isoflavone aglycone of the present invention is absorbed *in vivo*, food consumption is controlled, and an increase in body weight is controlled; and therefore, safe control of obesity is expected.
Nevertheless, compromise of immune function is not seen. Instead, immune function was even better than the group to which isoflavone aglycone was not added (comparative group 2).

### Example 3

Experiments were performed on male stroke-prone spontaneously hypertensive rats (SHRSP) in order to conduct experiments on males in this example.

### a) Experimental method

One-week acclimation was conducted using SHRSP rats (five weeks old) and then healthy SHRSP rats of the same body weight in each group (six weeks old) were reared as the samples. They were reared for 28 days beginning the day after acclimation on high-fat feed with the composition shown in Table 10 taken *ad libitum.* Tap water (3 ppm chlorine added) was provided *ad libitum.* Furthermore, the rearing facility environment was set so that the temperature and humidity are set at 23 ± 2°C and 55 ± 10%, the air conditioning system of ventilation (15 times/1 hour) is activated, and the lighting time was set from 8:00 a.m. to 8:00 p.m. Stainless steel metabolism cages as the rearing facility.

**Table 10**

| Composition of high-fat feed | Wt% |
|---|---|
| Lard | 25.0 |
| Corn oil | 11.0 |
| Milk casein | 24.0 |
| Corn starch | 15.5 |
| Granular sugar | 10.0 |
| Abicel (crystalline cellulose) | 3.0 |
| KC floc (cellulose powder) | 2.0 |
| Okanol (alpha-converted starch) | 1.0 |
| Vitamin mix (CLEA purified product) | 1.0 |
| Mineral mix (CLEA purified product) | 7.0 |

### 2) Administration of isoflavone aglycone

The SHRSP rats were grouped six rats each into a comparative group as well as experimental groups 1 and 2, and 5 wt% gum arabic solvent and isoflavone aglycone were orally administered gavage to each SHRSP rat of each group once a day using a stomach tube to obtain the doses shown in Table 11.

**Table 11**

| Administration group | Administration substance | Dose (mg/kg) |
|---|---|---|
| Comparative group | Solvent | -- |
| Experimental group 1 | Solvent + isoflavone aglycone | 20 |
| Experimental group 2 | Solvent + isoflavone aglycone | 40 |

### 3) Determination of body weight

Body weight of all SHRSP rats was determined the day before starting administration and once every day during the administration period.

### 4) Food consumption (amount of feed consumed)

Food consumption of all SHRSP rats was determined the day before starting administration and once every day during the administration period.

### 5) Blood pressure determinations

Blood pressure was determined on days 0, 7, 14, 21, and 28 during the period of administration of high-fat feed and isoflavone aglycone. Blood pressure determinations involved immobilizing the SHRSP rats on a holder and determining caudal arterial systolic blood pressure by the tail cuff method using an aneroid sphygmomanometer with temperature kept at 38 ± 1°C.

### 6) Urine collection, drawing of blood and determination of each determination item

Urine was collected by 24-hour urine specimens on days 0, 14, and 28 during the period of administration of high-fat feed and isoflavone aglycone. Urine NO₂/NO₃ and urine catecholamines (adrenaline, noradrenaline, and dopamine) were determined by the Griess method and HPLC, respectively. The animals were fasted for 24 hours after completing administration for 28 days, and autopsies were performed under ether anesthesia. Blood was drawn from the abdominal aorta; and serum total cholesterol, HDL-cholesterol, LDL-cholesterol, the cholesterol ester ratio, triglycerides, and free fatty acids were determined by the enzyme method. Furthermore, posterior abdominal wall fat, mesenteric fat, perinephric fat, and periepididymal fat were excised in order to determine *in vivo* fat and weight per 100 g body weight was determined.

### 7) Statistical treatment of data

Determination results for each group are represented by the mean ± standard deviation for each determination item, and significant difference tests were conducted using the Student's t method. The tests were two-sided tests, and the level of significance was 5 and 1%.

### b) Experimental results

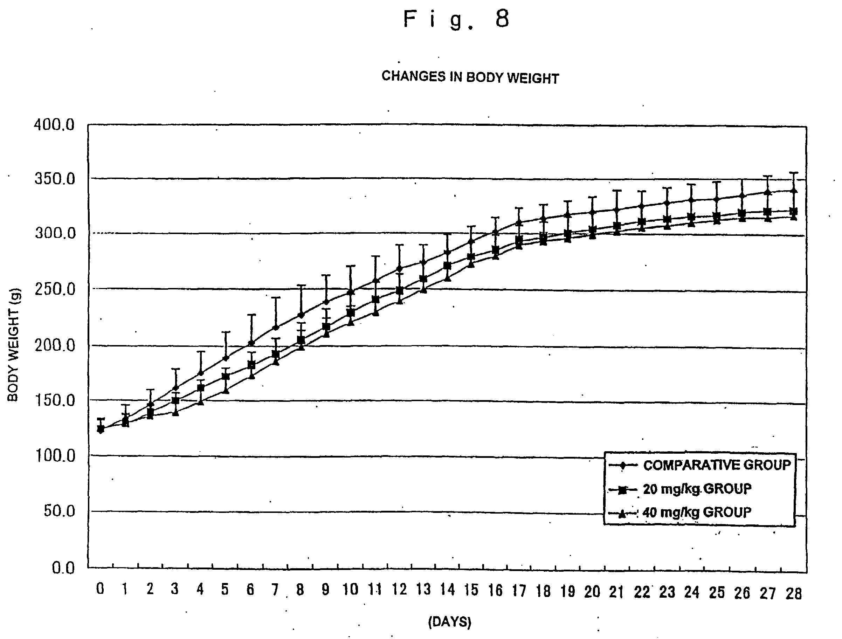

1) Observation of general condition
General condition was observed throughout the experimental period, but there were no particular anomalies.
2) The results of determinations of body weight are as shown in Table 12 and Figure 8.

**Table 12**

| Changes in Body Weight (g) | | | | |
|---|---|---|---|---|
| (day) | 0 | | 1 | 2 3 |
| Comparative group (n=6) | 123.2±11.30 | 134.0±11.92 | 146.8±13.35 | 16.20±17.35 |
| 20 mg/kg group (n=6) | 124.4±8.08 | 129.0±8.60 | 139.7±7.06 | 150.0±7.25 |
| 40 mg/kg group (n=6) | 124.6±9.25 | 129.3±9.49 | 136.6±9.40 | 140.0±9.48* |

| (day) | 4 | 5 | 6 | 7 |
|---|---|---|---|---|
| Comparative group (n=6) | 175.4±19.41 | 189.5±22.40 | 203.3±24.25 | 216.6±26.49 |
| 20 mg/kg group (n=6) | 161.6±7.11 | 172.4±7.91 | 182.6±11.80 | 192.6±14.26 |
| 40 mg/kg group (n=6) | 149.5±12.92* | 160.1±14.57* | 173.2±13.69* | 186.3±13.52* |

| (day) | 8 | 9 | 10 | 11 |
|---|---|---|---|---|
| Comparative group (n=6) | 227.6±25.98 | 239.1±23.93 | 247.4±23.12 | 257.8±22.08 |
| 20 mg/kg group (n=6) | 205.2±15.42 | 216.8±15.53 | 229.0±16.47 | 241.2±14.87 |
| 40 mg/kg group (n=6) | 199.0±14.47* | 210.5±14.14* | 221.0±13.84* | 230.0±10.86* |

| (day) | 12 | 13 | 14 | 15 |
|---|---|---|---|---|
| Comparative group (n=6) | 268.7±21.05 | 274.0±15.93 | 283.5±14.97 | 293.0±13.74 |
| 20 mg/kg group (n=6) | 248.7±15.32 | 259.5±15.38 | 271.3±13.80 | 279.7±12.54 |
| 40 mg/kg group (n=6) | 239.8±10.74* | 249.9±10.23* | 260.4±10.50* | 273.2±7.86* |

| (day) | 16 | 17 | 18 | 19 |
|---|---|---|---|---|
| Comparative group (n=6) | 302.0±12.91 | 311.0±12.68 | 314.5±12.54 | 317.9±12.50 |
| 20 mg/kg group (n=6) | 286.1±12.37 | 293.7±14.02* | 296.8±14.27* | 300.9±14.74 |
| 40 mg/kg group (n=6) | 280.5±9.03** | 290.3±7.58** | 293.8±7.54** | 296.2±6.97** |

| (day) | 20 | 21 | 22 | 23 |
|---|---|---|---|---|
| Comparative group (n=6) | 320.2±14.07 | 322.6±17.61 | 325.9±13.54 | 328.8±13.63 |
| 20 mg/kg group (n=6) | 304.5±14.71 | 308.2±14.99 | 311.8±14.85 | 314.1±14.75 |
| 40 mg/kg group (n=6) | 299.3±6.62* | 302.6±6061* | 305.9±6.42* | 307.8±5.76* |

| (day) | 24 | 25 | 26 | 27 |
|---|---|---|---|---|
| Comparative group (n=6) | 331.5±13.91 | 332.7±14.67 | 335.9±14.51 | 339.3±14.56 |
| 20 mg/kg group (n=6) | 316.1±14.35 | 317.2±15.05 | 320.0±15.07 | 321.5±15.76 |
| 40 mg/kg group (n=6) | 310.4±5.88* | 312.9±5.38* | 315.3±5.73* | 315.5±6.00** |

| (day) | 28 | | | |
|---|---|---|---|---|
| Comparative group (n=6) | 340.7±16.07 | | | |
| 20 mg/kg group (n=6) | 322.1±17.12 | | | |
| 40 mg/kg group (n=6) | 317.0±5.99* | | | |

| | | | | |
|---|---|---|---|---|
| (Mean ± standard deviation: tests of significant difference to the comparative group. *:*p* <0.05, | | | | |
| ****: *p* < 0.01) | | | | |

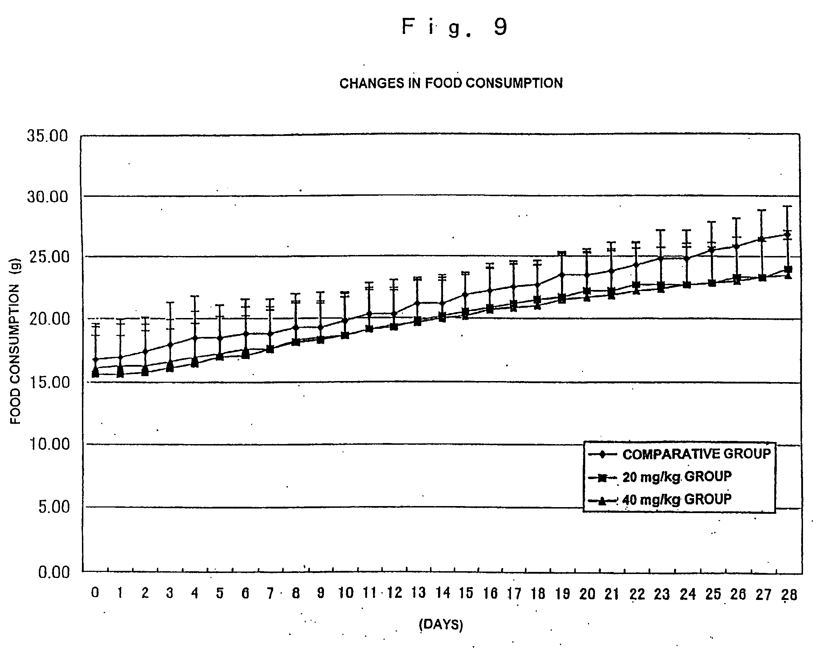

3) The results of determining food consumption are as shown in Table 13 and Figure 9.

**Table 13**

| Changes in Food Consumption (g) | | | | |
|---|---|---|---|---|
| (day) | 0 | 1 | 1 | 2 3 |
| Comparative group (n=6) | 16.83±2.79 | 17.00±2.97 | 17.50±2.59 | 18.00±3.29 |
| 20 mg/kg group (n=6) | 15.67±3.01 | 15.67±3.01 | 15.83±3.25 | 16.17±3.06 |
| 40 mg/kg group (n=6) | 16.17±3.19 | 16.33±3.27 | 16.33±3.27 | 16.67±3.27 |

| (day) | 4 | 5 | 6 | 7 |
|---|---|---|---|---|
| Comparative group (n=6) | 18.50+3.27 | 18.50±2.59 | 18.83±2.71 | 18.83±2.71 |
| 20 mg/kg group (n=6) | 16.50±3.15 | 17.00±3.16 | 17.17±3.06 | 17.67±3.01 |
| 40 mg/kg group (n=6) | 17.00±3.58 | 17.33±3.72 | 17.67±3.27 | 17.67±3.27 |

| (day) | 8 | 9 | 10 | 11 |
|---|---|---|---|---|
| Comparative group (n=6) | 19.33±2.07 | 19.33±2.07 | 19.83±2.23 | 20.33±2.07 |
| 20 mg/kg group (n=6) | 18.17±3.06 | 18.33±2.88 | 18.67±3.01 | 19.17±3.06 |
| 40 mg/kg group (n=6) | 18.33±3.61 | 18.50±3.56 | 18.67±3.27 | 19.17±3.66 |

| (day) | 12 | 13 | 14 | 15 |
|---|---|---|---|---|
| Comparative group (n=6) | 20.33±2.07 | 21.17±1.83 | 21.17±1.83 | 21.83±1.83 |
| 20 mg/kg group (n=6) | 19.33±2.88 | 19.83±3.25 | 20.17±3.06 | 20.50±3.15 |
| 40 mg/kg group (n=6) | 19.50±3.56 | 19.67±3.56 | 20.00±3.46 | 20.17±3.31 |

| (day) | 16 | 17 | 18 | 19 |
|---|---|---|---|---|
| Comparative group (n=6) | 22.17±1.83 | 22.50±1.87 | 22.67±1.63 | 23.50±1.87 |
| 20 mg/kg group (n=6) | 20.83±3.25 | 21.17±3.43 | 21.50±3.15 | 21.67±3.39 |
| 40 mg/kg group (n=6) | 20.67±3.72 | 20.83±3.76 | 21.00±3.29 | 21.50±3.73 |

| (day) | 20 | 21 | 22 | 23 |
|---|---|---|---|---|
| Comparative group (n=6) | 23.50±1.87 | 23.83±2.32 | 24.33±1.86 | 24.83±2.32 |
| 20 mg/kg group (n=6) | 22.17+3.43 | 22.17±3.43 | 22.67±3.44 | 22.67±3.08 |
| 40 mg/kg group (n=6) | 21.67±3.61 | 21.83±3.60 | 22.17±3.49 | 22.33±3.39 |

| (day) | 24 | 25 | 26 | 27 |
|---|---|---|---|---|
| Comparative group (n=6) | 24.83±2.32 | 25.50±2.35 | 25.83±2.32 | 26.50±2.35 |
| 20 mg/kg group (n=6) | 22.67±3.08 | 22.83±2.93 | 23.33±3.27 | 23.33±3.27 |
| 40 mg/kg group (n=6) | 22.67±3.39 | 22.83±3.31 | 23.00±2.97 | 23.33±2.94 |

| (day) | 28 | | | |
|---|---|---|---|---|
| Comparative group (n=6) | 26.83±2.32 | | | |
| 20 mg/kg group (n=6) | 24.00±3.15 | | | |
| 40 mg/kg group (n=6) | 23.50±2.95 | | | |
| (Mean ± standard deviation: tests of significant difference to the comparative group. *:*p*<0.05, **:*p*<0.01) | | | | |

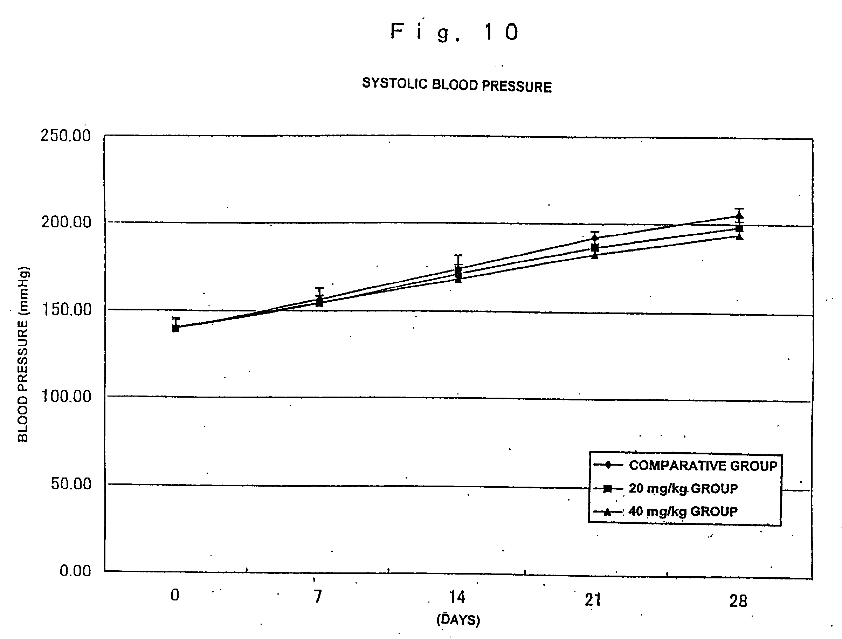

4) The results of determining systolic blood pressure are as shown in Table 14 and Figure 10.

**Table 14**

| Systolic blood pressure (mmHg) | | | | |
|---|---|---|---|---|
| (day) | 0 | 7 | 14 | 21 |
| Comparative group (n=6) | 140.20±4.92 | 156.70±6.12 | 173.70±7.87 | 192.00±3.74 |
| 20 mg/kg group (n=6) | 139.80±5.34 | 154.30±4.32 | 171.00±5.10 | 186.50±5.05 |
| 40 mg/kg group (n=6) | 140.70±5.50 | 154.70±4.23 | 168.00±6.54 | 182.5±6.25* |

| (day) | 28 | | | |
|---|---|---|---|---|
| Comparative group (n=6) | 205.50±4.04 | | | |
| 20 mg/kg group (n=6) | 198.30±6.09* | | | |
| 40 mg/kg group (n=6) | 193.80±8.04** | | | |

| | | | | |
|---|---|---|---|---|
| (Mean ± standard deviation; tests of significant difference to comparative group, *:p<0.05, | | | | |
| **:p<0.01) | | | | |

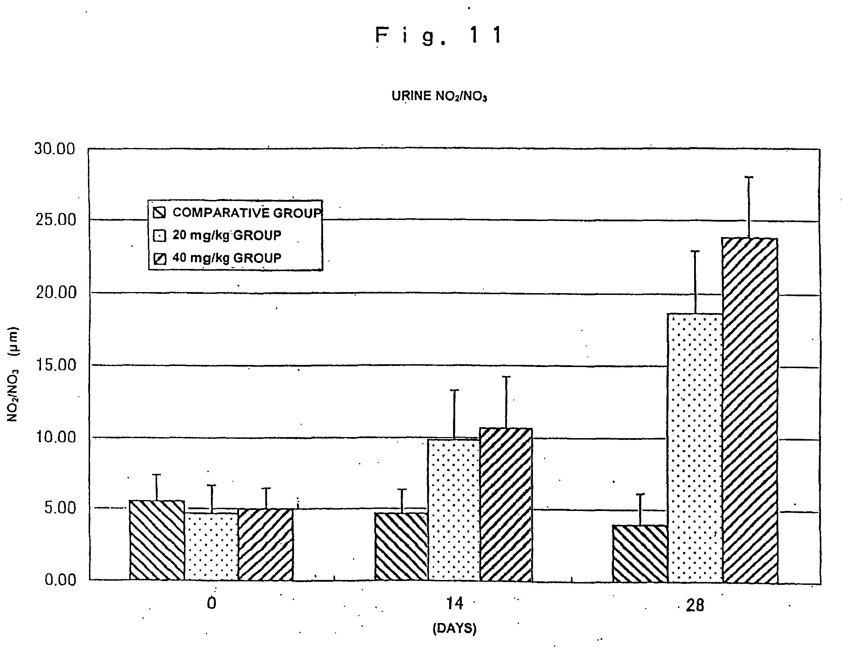

5) The results of determining urine NO₂/NO₃ are shown in Table 15 and Figure 11.

**Table 15**

| Urine NO₂/NO₃ (µM) | | | |
|---|---|---|---|
| (day) | 0 | 14 | 28 |
| Comparative group (n=6) | 5.50±1.87 | 4.67±1.63 | 4.00±2.10 |
| 20 mg/kg group (n=6) | 4.67±1.97 | 9.83±3.43* | 18.67±4.23* |
| 40 mg/kg group (n=6) | 5.00±1.41 | 10.67±3.56** | 23.83+4.17** |

| | | | |
|---|---|---|---|
| (Mean ± standard deviation; tests of significant difference to comparative group, *:p<0.05, | | | |
| **:p<0.01) | | | |

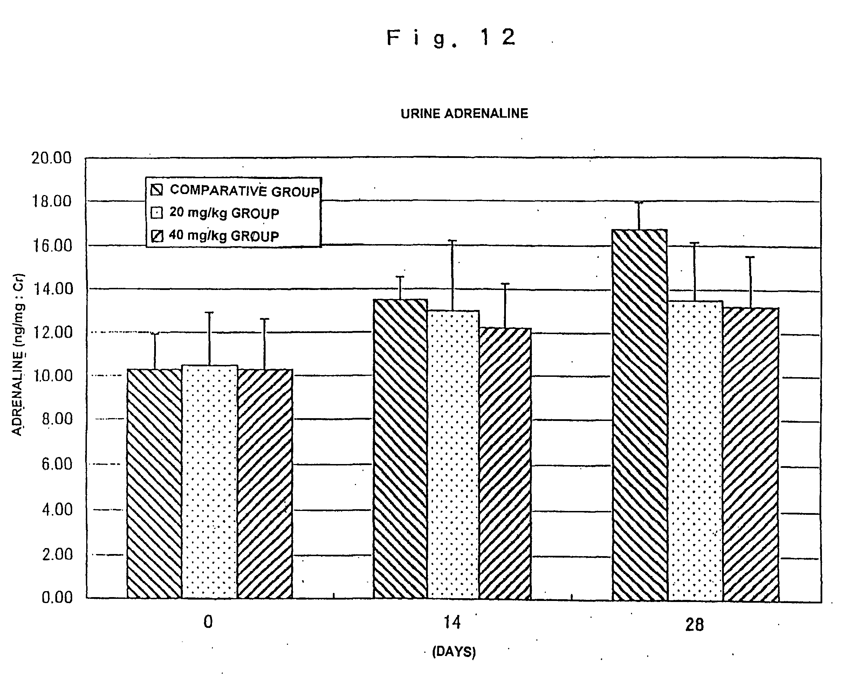

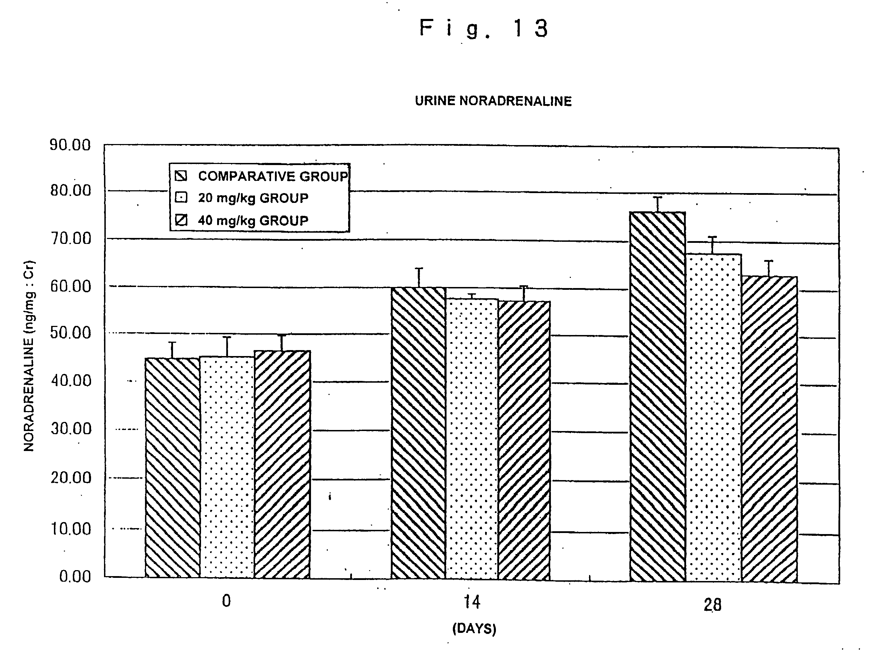

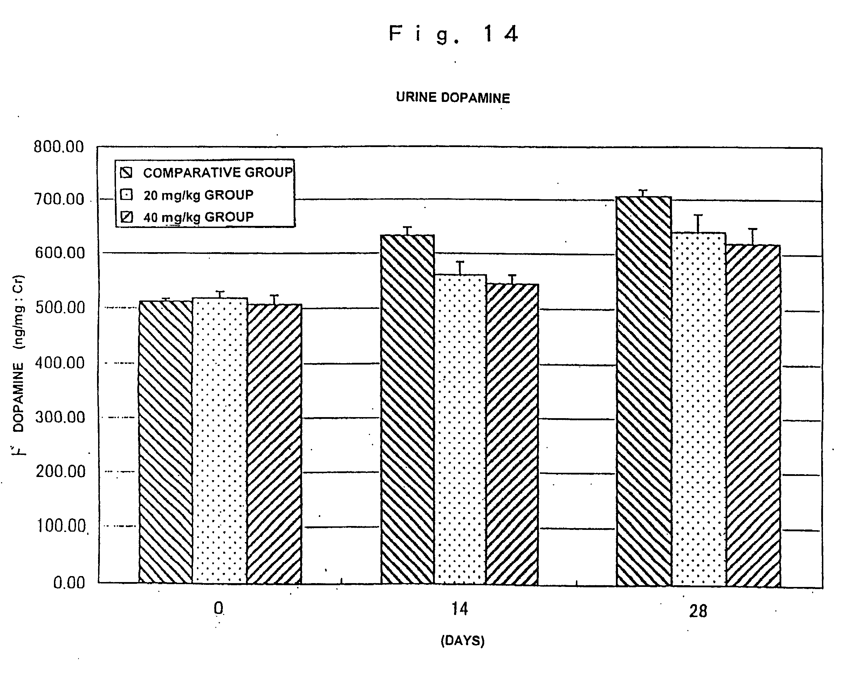

6) The determination results on urine catecholamines (adrenaline, noradrenaline, and dopamine) are as shown in Tables 16, 17, and 18 and Figure 12, 13, and 14.

**Table 16**

| Urine Adrenaline (ng/mg: Cr) | | | |
|---|---|---|---|
| (day) | 0 | 14 | 28 |
| Comparative group (n=6) | 10.30±1.63 | 13.50±1.05 | 16.70±1.21 |
| 20 mg/kg group (n=6) | 10.50±2.43 | 13.00±3.22 | 13.50±2.66* |
| 40 mg/kg group (n=6) | 10.30±2.34 | 12.20±2.04 | 13.20±2.32* |

| | | | |
|---|---|---|---|
| (Mean ± standard deviation; tests of significant difference to comparative group, *:p<0.05, **:p<0.01) | | | |

**Table 17**

| Urine Noradrenaline (ng/mg: Cr) | | | |
|---|---|---|---|
| (day) | 0 | 14 | 28 |
| Comparative group (n=6) | 44.80±3.43 | 60.00±3.95 | 76.00±3.03 |
| 20 mg/kg group (n=6) | 45.20±4.07 | 57.70±1.03 | 67.50±3.62** |
| 40 mg/kg group (n=6) | 46.50±3.15 | 57.30±3.20 | 63.00±3.16** |

| | | | |
|---|---|---|---|
| (Mean ± standard deviation; tests of significant difference to comparative group, *:p<0.05 , **:p<0.01) | | | |

**Table 18**

| Urine Dopamine (ng/mg: Cr) | | | |
|---|---|---|---|
| (day) | 0 | 14 | 28 |
| Comparative group (n=6) | 512.30±5.43 | 632.80±15.07 | 707.20±12.35 |
| 20 mg/kg group (n=6) | 518.70±11.83 | 561.20±22.79** | 640.70±32.95** |
| 40 mg/kg group (n=6) | 507.30±16.78 | 545.30±16.29** | 619.00±29.35** |

| | | | |
|---|---|---|---|
| (Mean ± standard deviation; tests of significant difference to comparative group, *:p<0.05, **:p<0.01) | | | |

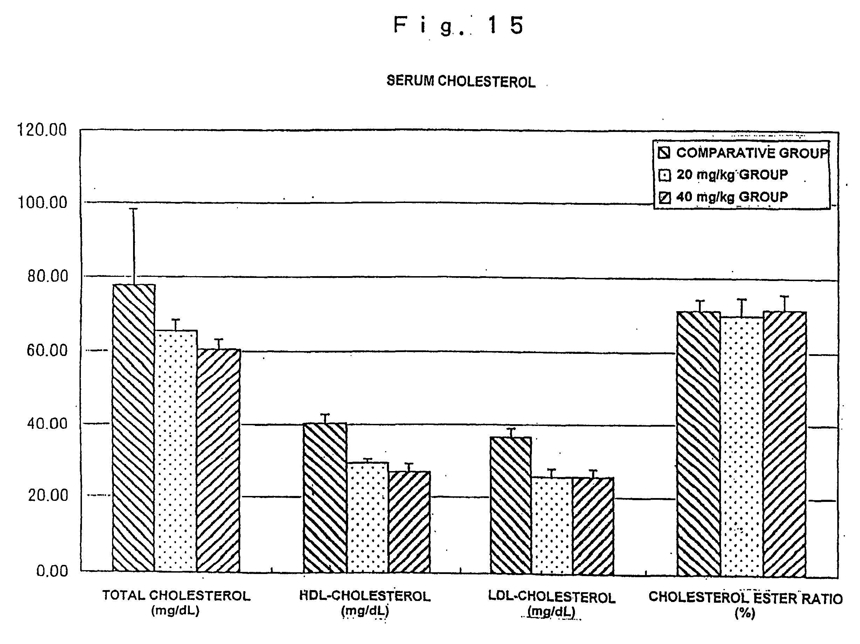

7) The results of determining serum total cholesterol, HDL-cholesterol, LDL-cholesterol, and the cholesterol ester ratio are as shown in Table 19 and Figure 15.

**Table 19**

| Serum Total Cholesterol, HDL-cholesterol, LDL-cholesterol, Cholesterol Ester Ratio | | | | |
|---|---|---|---|---|
| | Total cholesterol (mg/dL) | HDL-cholesterol (mg/dL) | LDL-cholesterol (mg/dL) | Cholesterol ester ratio (%) |
| Comparative group (n=6) | 77.78±20.64 | 40.32±2.69 | 36.57±2.36 | 71.17±3.06 |
| 20 mg/kg group (n=6) | 65.47±3.01 | 29.20±1.14** | 25.47±2.20** | 69.83±4.83 |
| 40 mg/kg group (n=6) | 60.58±2.53 | 26.68±2.28** | 25.45±2.07** | 71.50±4.04 |

| | | | | |
|---|---|---|---|---|
| (Mean ± standard deviation; tests of significant difference to comparative group, *:p<0.05, **:p<0.01) | | | | |

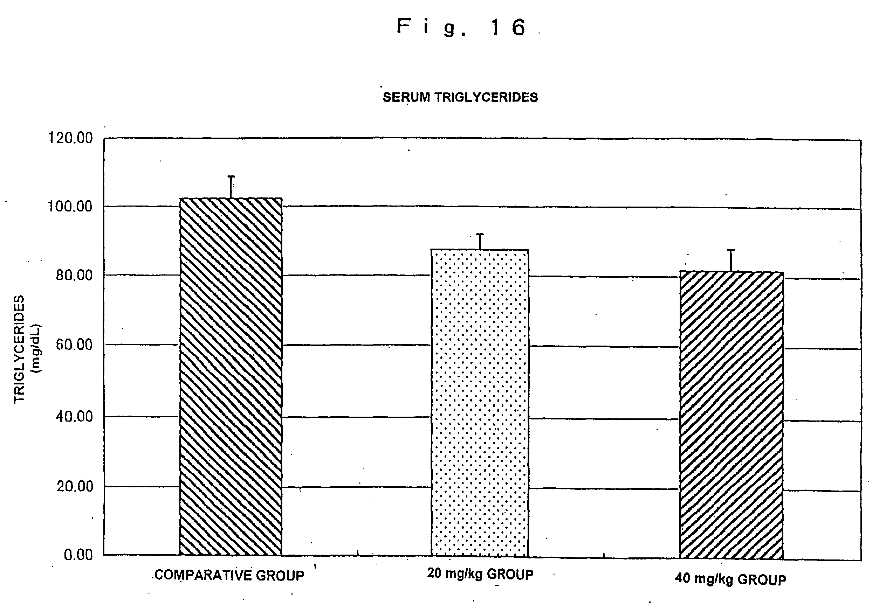

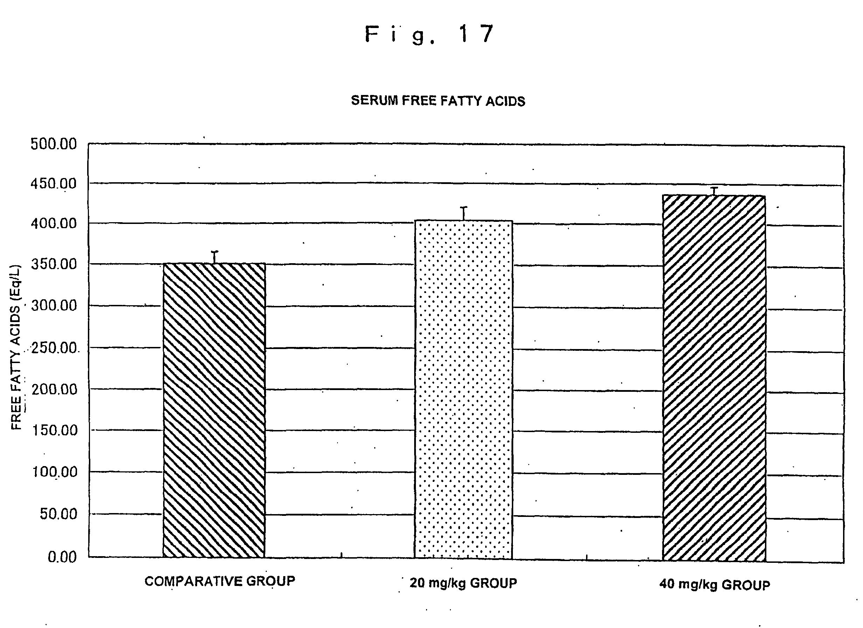

8) The results of determining serum triglycerides and free fatty acids are as shown in Table 20 and Figures 16 and 17.

**Table 20**

| Serum Triglycerides, Free Fatty Acids | | |
|---|---|---|
| | Triglycerides (mg/dL) | Free fatty acids (µEq/L) |
| Comparative group (n=6) | 102.32±6.42 | 351.00±13.80 |
| 20 mg/kg group (n=6) | 87.57±4.52** | 403.50±16.45** |
| 40 mg/kg group (n=6) | 81.73±6.22** | 436.17±9.79** |

| | | |
|---|---|---|
| (Mean ± standard deviation; Test of significant difference to comparative group *: p<0.05, **:p<0.01) | | |

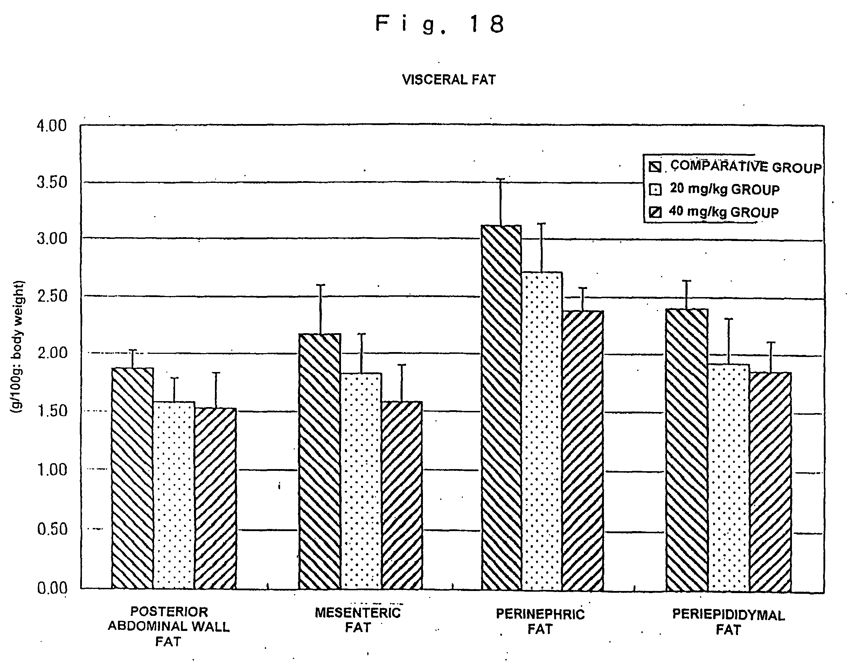

9) The results of determining posterior abdominal wall fat, mesenteric fat, perinephric fat, and periepididymal fat relating to *in vivo* fat are as shown in Table 21 and Figure 18.

**Table 21**

| Visceral Fat Weight (g/100 g body weight) | | | | |
|---|---|---|---|---|
| | Posterior abdominal wall fat | Mesenteric fat | Perinephric fat | Periepididymal fat |
| Comparative group (n=6) | 1.87±0.16 | 2.17±0.43 | 3.12±0.41 | 2.40±0.25 |
| 20 mg/kg group (n=6) | 1.58±0.20* | 1.82±0.35 | 2.72±0.42 | 1.92±0.40* |
| 40 mg/kg group (n=6) | 1.53±0.30* | 1.58±0.32* | 2.38±0.20 | 1.85±0.27 |

| | | | | |
|---|---|---|---|---|
| (Mean ± standard deviation; Test of significant difference to comparative group *: p<0.05, **:p<0.01) | | | | |

### c) Evaluation

As seen from the changes in body weight in Table 12 and Figure 8, when the comparative group and experimental groups 1 and 2 are compared in terms of changes in body weight of SHRSP rats during the experimental period, the same control of an increase in body weight as with female rats is seen with male SHRSP rats. It can, therefore, be said that an increase in body weight was controlled in male rats as well by administration of the anti-obesity material of the present invention. This tendency became stronger with an increase in the isoflavone aglycone dose. This is nothing short of isoflavone aglycone having excellent absorption *in vivo*.

If the comparative group is regarded as rats of normal body weight, weighing less can be regarded as a tendency toward losing weight. When evaluated from this standpoint, an increase in body weight in experimental groups 1 and 2 was inhibited when compared to the comparative group. When explained in further detail, experimental group 1 where the isoflavone aglycone dose was 20 mg/kg was significantly low on days 17, 18, and 20 - 24, and as a result, an increase in body weight of 5% was controlled; while experimental group 2, where the isoflavone aglycone dose was 40 mg/kg, was significantly low on days 3 - 24, and as a result, an increase in body weight of 7% was controlled.

As seen from the changes in food consumption in Table 13 and Figure 9, when the comparative group and experimental groups 1 and 2 are compared in terms of changes in food consumption of SHRSP rats during the experimental period, significant differences were not seen; however, there was a tendency toward control of food consumption in experimental groups 1 and 2 administered isoflavone aglycone.

As seen from the changes in systolic blood pressure in Table 14 and Figure 10, when the comparative group and experimental groups 1 and 2 are compared in terms of systolic blood pressure of SHRSP rats during the experimental period, although a reduction was not seen on days 7 and 14, a significant reduction was seen on days 21 and 28. When explained in further detail, a significantly low value was obtained on day 28 in experimental group 1 where the isoflavone aglycone dose was 20 mg/kg; while a significantly low value was obtained on days 21 and 28 in experimental group 2 where the isoflavone aglycone dose was 40 mg/kg.

As seen from the changes in urine NO₂/NO₃ in Table 15 and Figure 11, when the comparative group and experimental groups 1 and 2 are compared in terms of changes in urine NO₂/NO₃ of SHRSP rats during the experimental period, the value was significantly high both on days 14 and 28 for both experimental groups 1 and 2. This confirms that the isoflavone aglycone administered in both experimental groups 1 and 2 has estrogen-like activity, and therefore, eNOS (endothelial-type NO-synthesizing enzyme) activity is increased and NO is produced.

As seen from the changes in urine catecholamines (adrenaline, noradrenaline, and dopamine) in Tables 16, 17 and 18 and Figures 12, 13, and 14, when the comparative group and experimental groups 1 and 2 are compared in terms of changes in adrenaline and noradrenaline of SHRSP rats during the experimental period, the values were significantly low both on day 28 in both experimental groups 1 and 2, while the dopamine level was significantly low both on days 14 and 28 in both experimental groups 1 and 2. This indicates that a reduction in blood pressure can be expected because there is a significant reduction in urine catecholamines (adrenaline, noradrenaline, and dopamine).

As seen from the changes in the serum total cholesterol, HDL-cholesterol, LDL-cholesterol, and cholesterol ester ratio in Table 19 and Figure 15, when the comparative group and experimental groups 1 and 2 are compared in terms of changes in HDL-cholesterol and LDL-cholesterol of SHRSP rats during the experimental period, significantly low values were seen in both experimental groups 1 and 2. Furthermore, there was a tendency toward a reduction in serum total cholesterol in both experimental groups 1 and 2, making it clear that isoflavone aglycone is effective not only in regulating blood pressure but also in promoting fat metabolism, etc., even in males. Furthermore, it is healthier to not show a significant difference in the cholesterol ester ratio, and there was no significant difference, making it clear that isoflavone aglycone can retain help in maintaining good health while controlling an increase in body weight and elevation of blood pressure.

As seen from the changes in serum triglycerides and free fatty acids in Table 20 and Figures 16 and 17, when the comparative group and experimental groups 1 and 2 are compared in terms of changes in the serum triglycerides and free fatty acids of SHRSP rats during the experimental period, both values were significantly low in both experimental groups 1 and 2. It is clear that by significantly lowering serum triglycerides as described above, isoflavone aglycone is effective not only in regulating blood pressure, but also in promoting fat metabolism, even in males. Furthermore, it is clear that by significantly increasing serum free fatty acids in both experimental groups 1 and 2, isoflavone aglycone is effective not only in regulating blood pressure but also in decomposing fats and promoting fat metabolism, even in males.

As seen from the changes in posterior abdominal wall fat, mesenteric fat, perinephric fat, and periepididymal fat relating to *in vivo* fat in Table 21 and Figure 18, when the comparative group and experimental groups 1 and 2 are compared in terms of changes in posterior abdominal wall fat, mesenteric fat, perinephric fat, and periepididymal fat of SHRSP rats during the experimental period, posterior abdominal wall fat was significantly low in both experimental groups 1 and 2, mesenteric fat was significantly low in experimental group 2 with a high isoflavone aglycone dose, and periepididymal fat was significantly low in experimental group 1 with a low isoflavone aglycone dose. It was made clear that by significantly reducing *in vivo* fats, isoflavone aglycone is effective not only in regulating blood pressure but also in promoting fat metabolism, etc., even in males.

Moreover, good results were obtained by oral administration of the administration substance gavage using a stomach tube in Examples 1, 2 and 3. Thus, oral ingestion is facilitated and the above-described excellent results can be realized by safe absorption from the digestive tract by forming the anti-obesity material of the present invention into an oral supplement form in, for instance, capsules, tablets, granules, powder, etc., that can be easily taken orally.

Furthermore, the present invention is not limited to the above-described embodiments and examples, and it can be changed as needed. In addition to oral administration, the anti-obesity material of the present invention can be absorbed *in vivo* using drops.

## Claims

1. An anti-obesity material, **characterized in that** said anti-obesity material contains isoflavone aglycone and/or isoflavone glycoside.

2. The anti-obesity material according to claim 1, **characterized in that** said isoflavone aglycone and isoflavone glycoside is a material derived from grains.

3. The anti-obesity material according to claim 2, **characterized in that** said grain-derived material is produced by performing fermentation of grains by koji mold so as to decompose protein and then by performing hydrolysis.

4. The anti-obesity material according to claim 3, **characterized in that** said grains are pulse crops.

5. The anti-obesity material according to claim 3 or 4, **characterized in that** said isoflavone aglycone is produced by further performing concentration of a material produced by hydrolysis.

6. The anti-obesity material according to claim 5, **characterized in that** said isoflavone aglycone contains at least 70 wt% daizein.

7. The anti-obesity material according to any one of claims 1 through 6, **characterized in that** said anti-obesity material is formed into an oral supplement.
